# EUROPÄISCHE PATENTSCHRIFT

(11) **EP 3 278 093 B1**
(45) Veröffentlichungstag und Bekanntmachung des Hinweises auf die Patenterteilung: **02.10.2019**
(21) Anmeldenummer: 16712326.4
(22) Anmeldetag: 24.03.2016
(51) Int. Cl.: G01N 27/417, F01N 11/00, F02D 41/14, F02D 41/22, G01N 27/419

(54) **VERFAHREN ZUM BETREIBEN EINER SONDE**
METHOD TO CONTROL A SENSOR
PROCÉDÉ DE CONTRÔLER D'UN CAPTEUR

(30) Priorität: 01.04.2015 DE 102015205971
(43) Veröffentlichungstag der Anmeldung: 07.02.2018
(73) Patentinhaber: Continental Automotive GmbH, 30165 Hannover (DE)
(72) Erfinder: LEMIRE, Bertrand, 84069 Schierling (DE); BENTNER, Johannes, 93080 Pentling (DE); KOLBECK, Sabrina, 93458 Eschlkam (DE); KILINC, Muammer, 93055 Regensburg (DE)
(86) Internationale Anmeldenummer: PCT/EP2016/056677
(87) Internationale Veröffentlichungsnummer: WO 2016/156254

(56) Entgegenhaltungen:
- WO-A1-2010/020641
- DE-B3-102008 024 177
- US-B1- 6 290 829

## Beschreibung

Die Erfindung betrifft ein Verfahren zum Betreiben einer Sonde mit einer gepumpten Referenzzelle. Sonden zur Erfassung einer Konzentration eines Gases aus einem Gasgemisch werden heutzutage vielseitig eingesetzt. Insbesondere in modernen Fahrzeugen können Sonden zum Einsatz kommen, um beispielsweise eine Konzentration eines Gases im Verbrennungsabgas zu ermitteln.

Die WO 2010/020641 A1 betrifft ein Verfahren und eine Vorrichtung zum Überprüfen eines Abgassensors.

Die US 6,290,829 B1 betrifft einen Gassensor
Aus der DE 10 2008 024 177 B3 ist ein Verfahren, eine Vorrichtung und ein System zur Diagnose eines NOx-Sensors für eine Brennkraftmaschine bekannt.

Die Aufgabe, die der Erfindung zugrunde liegt, ist es, ein Verfahren zum Betreiben einer Sonde mit einer gepumpten Referenzzelle zu schaffen, das zu einem zuverlässigen und präzisen Betrieb der Sonde beiträgt.

Die Aufgabe wird gelöst durch den unabhängigen Patentanspruch. Vorteilhafte Ausgestaltungen sind in den Unteransprüchen gekennzeichnet.

Die Erfindung zeichnet sich aus durch ein Verfahren zum Betreiben einer Sonde mit einer gepumpten Referenzzelle . Es wird ein erster Zeitpunkt ermittelt, in dem ein an der Referenzzelle anliegender Pumpstrom angeschaltet ist. Abhängig von dem ersten Zeitpunkt wird zumindest ein erster Spannungskennwert innerhalb eines vorgegebenen ersten Zeitfensters ermittelt, der repräsentativ ist für eine an der Referenzzelle anliegende Spannung. Es wird der an der Referenzzelle anliegende Pumpstrom in einem zweiten Zeitpunkt abgeschaltet. Ein dritter Zeitpunkt wird ermittelt, in dem ein an der Referenzzelle anliegender Pumpstrom abgeschaltet ist. Abhängig von dem dritten Zeitpunkt wird zumindest ein zweiter Spannungskennwert innerhalb eines vorgegebenen zweiten Zeitfensters ermittelt, der repräsentativ ist für die an der Referenzzelle anliegende Spannung. Abhängig von dem zumindest einen ersten Spannungskennwert und dem zumindest einen zweiten Spannungskennwert wird ein Diagnosekennwert ermittelt, der repräsentativ ist für eine Impedanz der Referenzzelle.

Dies hat den Vorteil, dass eine elektrische Diagnose einer Impedanz der Referenzzelle durchgeführt werden kann. Insbesondere kann es sich bei der Diagnose um eine Eigendiagnose der Sonde im Betrieb der Sonde handeln. Hierzu kann auf einen entsprechenden Vorhalt bei Auslegung des Pumpstroms verzichtet werden, also eine Amplitude des Pumpstroms betragsmäßig unverändert hoch dimensioniert sein, wodurch eine Referenzluftvergiftung (auch "chemical shift down" genannt) vermieden werden kann. Ferner kann die Eigendiagnose insbesondere frei von aufwändigen Rahmenbedingungen erfolgen, die beispielsweise lediglich durch Demontage der Sonde zu erreichen wären. Dies ermöglicht es zum Beispiel eine defekte Sonde mit zu stark gealterter Zellimpedanz im Feld zu detektieren.

Bei der Sonde handelt es sich insbesondere um eine Sonde zur Ermittlung einer Gaskonzentration aus einem Gasgemisch. Insbesondere wird die Sonde hierzu amperometrisch betrieben.

Beispielsweise handelt es sich bei der Sonde um einen Stickoxidsensor oder einen Sauerstoffsensor.

Der erste Zeitpunkt entspricht beispielsweise einem Startzeitpunkt einer Eigendiagnose der Sonde. Der zweite Zeitpunkt folgt in diesem Fall zeitlich auf den ersten Zeitpunkt. Alternativ hierzu entspricht der zweite Zeitpunkt einem Startzeitpunkt der Eigendiagnose der Sonde, wobei der erste Zeitpunkt zeitlich auf den zweiten Zeitpunkt folgt. Dies ist insbesondere der Fall, wenn sichergestellt ist, dass die an der Referenzzelle anliegende Spannung repräsentativ ist für eine gültige Eigendiagnose der Sonde.

Die durch den Diagnosekennwert repräsentierte Impedanz der Referenzzelle kann einen Fehler eines Messergebnisses der Sonde bedingen. In diesem Zusammenhang kann abhängig von dem zumindest einen ersten Spannungskennwert und dem zumindest einen zweiten Spannungskennwert eine Bewertung erfolgen, die in den Diagnosekennwert zur Eigendiagnose einfließt. Der Diagnosekennwert kann in diesem Zusammenhang auch als Maß für eine Empfindlichkeit der Sonde dienen, beispielsweise als Maß für eine Stickoxid- und/oder Sauerstoffempfindlichkeit der Sonde und/oder als ein Maß für einen Zustand der Sonde, wie beispielsweise "gut" oder "schlecht".

In einer vorteilhaften Ausgestaltung wird zwischen dem zweiten Zeitpunkt und dem dritten Zeitpunkt eine Wartephase für eine vorgegebene erste Zeitspanne durchgeführt. Dies hat den Vorteil dass der zumindest eine in dem zweiten vorgegebenen Zeitfenster ermittelte zweite Spannungskennwert im Wesentlichen stabil ist, das heißt, dass die an der Referenzzelle anliegende Spannung im Wesentlichen in einem eingeschwungenen Zustand ist.

In vorteilhafter Weise trägt dies zu einer hohen Präzision des zumindest einen ermittelten zweiten Spannungskennwerts bei.

In einer weiteren vorteilhaften Ausgestaltung wird der an der Referenzzelle anliegende Pumpstrom in einem vierten Zeitpunkt angeschaltet. Der vierte Zeitpunkt folgt zeitlich auf den dritten Zeitpunkt. In vorteilhafter Weise wird so dazu beigetragen, dass die an der Referenzzelle anliegende Spannung bei Ermittlung des mindestens einen zweiten Spannungskennwerts bis zu dem vierten Zeitpunkt stabil ist.

In einer weiteren vorteilhaften Ausgestaltung wird ein fünfter Zeitpunkt ermittelt, in dem ein an der Referenzzelle anliegender Pumpstrom angeschaltet ist. Der fünfte Zeitpunkt folgt zeitlich auf den vierten Zeitpunkt. Abhängig von dem fünften Zeitpunkt wird zumindest ein dritter Spannungskennwert innerhalb eines vorgegebenen dritten Zeitfensters ermittelt, der repräsentativ ist für die an der Referenzzelle anliegende Spannung. Dies ermöglicht eine Verifizierung, ob die an der Referenzzelle anliegende Spannung repräsentativ ist für eine gültige Eigendiagnose der Sonde.

In einer weiteren vorteilhaften Ausgestaltung wird zwischen dem vierten Zeitpunkt und dem fünften Zeitpunkt eine Wartephase für eine vorgegebene zweite Zeitspanne durchgeführt. Dies hat den Vorteil, dass der zumindest eine in dem dritten vorgegebenen Zeitfenster ermittelte dritte Spannungskennwert im Wesentlichen stabil ist, das heißt, dass die an der Referenzzelle anliegende Spannung im Wesentlichen in einem eingeschwungenen Zustand ist. In vorteilhafter Weise trägt dies zu einer hohen Präzision des zumindest einen ermittelten dritten Spannungskennwerts bei.

In einer weiteren vorteilhaften Ausgestaltung wird der Diagnosekennwert abhängig von dem zumindest einen dritten Spannungskennwert ermittelt. Dies dient einer besonders präzisen und zuverlässigen Ermittlung des Diagnosekennwerts.

In einer weiteren vorteilhaften Ausgestaltung umfasst ein Ermitteln des Diagnosekennwerts eine Ermittlung einer Differenz abhängig von den jeweiligen Spannungskennwerten. Dies ermöglicht eine einfache Ermittlung des Diagnosekennwerts. Beispielsweise umfasst die Ermittlung des Diagnosekennwerts ein Ermitteln der Differenz des zumindest einen ersten Spannungskennwerts und des zumindest einen zweiten Spannungskennwerts.

In einer weiteren vorteilhaften Ausgestaltung umfasst ein Ermitteln des Diagnosekennwerts eine Ermittlung eines Mittelwerts abhängig von den jeweiligen Spannungskennwerten. Dies ermöglicht eine besonders einfache Ermittlung des Diagnosekennwerts. Beispielsweise umfasst die Ermittlung des Diagnosekennwerts ein Ermitteln der Differenz jeweiliger Mittelwerte des zumindest einen ersten Spannungskennwerts und des zumindest einen zweiten Spannungskennwerts. Alternativ oder zusätzlich wird ein von der Ermittlung des Mittelwerts verschiedenes Signalfiltern durchgeführt.

In einer weiteren vorteilhaften Ausgestaltung umfasst ein Ermitteln des Diagnosekennwerts eine Ermittlung einer Standardabweichung abhängig von den jeweiligen Spannungskennwerten. Dies ermöglicht eine einfache Verifizierung, ob die an der Referenzzelle anliegende Spannung repräsentativ ist für eine gültige Eigendiagnose der Sonde. In diesem Zusammenhang wird zu einer präzisen und zuverlässigen Ermittlung des Diagnosekennwerts beigetragen. Beispielsweise wird hierzu der erste Spannungskennwert mit dem dritten Spannungskennwert verglichen, so dass ein Abdriften der an der Referenzzelle anliegenden Spannung während der Diagnose überprüft werden kann. Insbesondere wird dabei eine Differenz des ersten Spannungskennwerts und des dritten Spannungskennwerts ermittelt. Abhängig von dem Vergleich erfolgt dann beispielhaft eine Bewertung im Hinblick auf eine Gültigkeit der Eigendiagnose.

In einer weiteren vorteilhaften Ausgestaltung umfasst die Sonde eine Messzelle. Abhängig von dem Diagnosekennwert wird ein Sollspannungskennwert ermittelt, der repräsentativ ist für eine an der Messzelle anliegende Spannung zur Messung einer Gaskonzentration eines der Sonde zugeführten Gasgemischs. Ein an der Messzelle anliegender Strom wird derart gesteuert, dass sich die an der Messzelle anliegende Spannung auf den Sollspannungskennwert einstellt.

In vorteilhafter Weise ermöglicht dies eine Kompensation der Impedanz der Referenzzelle. Der an der Messzelle anliegende Strom ist in diesem Zusammenhang repräsentativ für die Gaskonzentration des der Sonde zugeführten Gasgemischs. Ein Ermitteln des Sollspannungskennwerts abhängig von dem Diagnosekennwert kann auch als Kompensation eines Sollwerts der Nernstspannung bezeichnet werden. Alternativ oder zusätzlich kann die Sonde insbesondere mehrere Messzellen umfassen, wobei abhängig von dem Diagnosekennwert beispielsweise eine gemeinsame oder separate Kompensation jeweiliger Sollwerte der Nernstspannungen durchgeführt wird.

In einer weiteren vorteilhaften Ausgestaltung ist die Sonde als Sauerstoffsensor ausgebildet oder umfasst einen solchen. Abhängig von dem Diagnosekennwert wird eine Sauerstoffkonzentration eines dem Sauerstoffsensor zugeführten Gasgemischs ermittelt. Beispielsweise erfolgt in diesem Zusammenhang ein Ermitteln von Korrektur-Parametern abhängig von dem Diagnosekennwert, so dass korrekte Betriebspunkte des Sauerstoffsensors eingestellt werden können und eine Veränderung der Impedanz der Referenzzelle kompensiert werden kann. Bei der ermittelten Sauerstoffkonzentration kann es sich insbesondere um eine korrigierte Sauerstoffkonzentration handeln.

In einer weiteren vorteilhaften Ausgestaltung ist die Sonde als Stickoxidsensor in einem Abgastrakt einer Verbrennungsmaschine angeordnet. Abhängig von dem Diagnosekennwert wird eine Stickoxidkonzentration eines dem Stickoxidsensor zugeführten Gasgemischs ermittelt. Ein Ermitteln des ersten Zeitpunkts wird abhängig von einem Betriebszustand der Verbrennungsmaschine durchgeführt. Der Betriebszustand der Verbrennungsmaschine, zu dem der erste Zeitpunkt ermittelt wird, kann beispielsweise eines umfassen aus: Teillast, Schub, Nachlauf und λ=1 Betriebspunkt. In vorteilhafter Weise trägt dies zu einer besonders präzisen und zuverlässigen Ermittlung des Diagnosekennwerts bei. Insbesondere ist bei den genannten Betriebszuständen davon auszugehen, dass eine im Wesentlichen stabile Spannung an der Referenzzelle anliegt.

Darüber hinaus erfolgt beispielsweise optional ein Ermitteln von Korrektur-Parametern abhängig von dem Diagnosekennwert, so dass korrekte Betriebspunkte des Stickoxidsensors eingestellt werden können und eine Veränderung der Impedanz der Referenzzelle kompensiert werden kann.

Ausführungsbeispiele sind im Folgenden anhand der schematischen Zeichnungen näher erläutert. Es zeigen:
- Figur 1: ein Verlauf einer Referenzspannung einer Referenzzelle einer Sonde in schematischer Darstellung,
- Figur 2: ein Ablaufdiagramm zum Betreiben der Sonde.

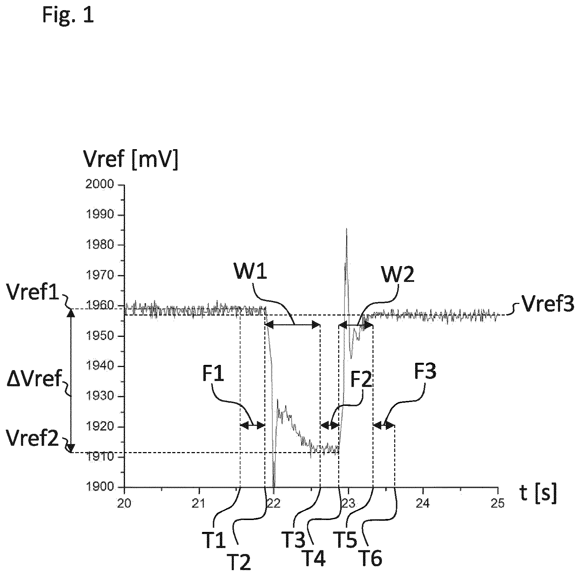

Sonden zur Erfassung einer Konzentration eines Gases aus einem Gasgemisch werden heutzutage vielseitig eingesetzt. Insbesondere in modernen Fahrzeugen können Sonden zum Einsatz kommen, um beispielsweise eine Restsauerstoffkonzentration im Verbrennungsabgas, und/oder eine Stickoxidkonzentration im Verbrennungsabgas zu ermitteln. Hierbei wird oftmals ein Teil der Sonde dem Verbrennungsabgas ausgesetzt, während an einem weiteren Teil der Sonde eine Sauerstoffreferenz anliegt. In diesem Zusammenhang kann die Sonde eine gepumpte Referenzzelle aufweisen, die abhängig von einem an der Referenzzelle anliegenden Pumpstrom die Sauerstoffreferenz bereitstellt. Figur 1 zeigt einen Verlauf einer Referenzspannung Vref einer solchen gepumpten Referenzzelle im Betrieb einer im Abgasstrang eines Fahrzeugs angeordneten amperometrisch betriebenen Stickoxidsonde, aufgetragen über die Zeit t. Die Referenzzelle stellt den Bezugspunkt für alle in der Sonde gemessenen Sauerstoffkonzentrationen dar. Hierzu wird eine Nernstspannung gemessen. Die Referenzzelle wird beispielsweise über einen pulsweiten-modulierten (PWM) Strom gesteuert.

In dem dargestellten Verlauf beträgt die Referenzspannung Vref über einen ersten Zeitpunkt T1 bis hin zu einem zweiten Zeitpunkt T2 in etwa 1960 mV. In diesem Zeitraum befindet sich die Referenzzelle in einem gepumpten Betriebszustand. Der gepumpte Betriebszustand kann durch das Anlegen eines Gleichstroms, als auch eines pulsweiten-modulierten Stromes an die Referenzzelle ausgeführt sein. In dem dargestellten Verlauf wird die Referenzspannung Vref insbesondere während der Ausschaltzeit des pulsweiten-modulierten Stromes gemessen, also nicht während eines Pulses des pulsweiten-modulierten Stromes. In diesem Zusammenhang kann der pulsweiten-modulierte Strom auch als Pumpstrom bezeichnet werden.

Zwischen dem zweiten Zeitpunkt T2 über einen dritten Zeitpunkt T3 hin zu einem vierten Zeitpunkt T4 schwankt die Referenzspannung Vref stark und pendelt sich schließlich bei etwa 1910 mV ein. In diesem Zeitraum befindet sich die Referenzzelle in einem ungepumpten Betriebszustand. Der pulsweiten-modulierte Strom kann dabei beispielsweise ein betragsmäßiges Minimum, beispielsweise 0 A annehmen.

Zwischen dem vierten Zeitpunkt T4 über einen fünften Zeitpunkt T5 hin zu einem sechsten Zeitpunkt T6 schwankt die Referenzspannung Vref zunächst stark und pendelt sich schließlich etwa bei dem Ausgangswert von 1960 mV ein. Die Referenzzelle befindet sich in diesem Zeitraum in dem gepumpten Betriebszustand.

Die Nernstspannung der Messkavitäten in der Sonde werden bevorzugt in einem stromlosen Zustand der Referenzzelle gemessen, also wenn ein PWM-Strom-Puls abgeklungen ist. Anderenfalls kann die Nernstspannung durch den ohmschen Spannungsabfall verfälscht sein. Dies hätte beispielsweise zur Folge, dass die Sonde in einem falschen Betriebspunkt laufen würde, welcher von der ursprünglichen Kalibration abweicht und demzufolge eine falsche Stickoxidkonzentration ausgeben würde.

Insbesondere kann dies der Fall sein, wenn die Referenzzelle neben dem rein Ohm'schen Anteil einen kapazitiven Anteil besitzt. Beispielsweise bei Referenzzellen, die Yttrium stabilisiertes Zirkonoxid aufweisen, kann eine Impedanz der Referenzelle über Alterung stark zunehmen. Abhängig von der Impedanz der Referenzzelle und der damit einhergehenden Abklingzeit nach Abschalten des PWM Pulses kann die Messung verfälscht werden.

Durch eine Messung einer Differenz ΔVref der Referenzspannung Vref zwischen dem gepumpten Betriebszustand der Referenzzelle und dem ungepumpten Betriebszustand der Referenzzelle wird nun im Rahmen einer Eigendiagnose der Sonde ermittelt, ob die Referenzspannung Vref im Wesentlichen vollständig relaxiert oder aufgrund einer zu hohen Impedanz der Referenzzelle ein Spannungsversatz vorliegt.

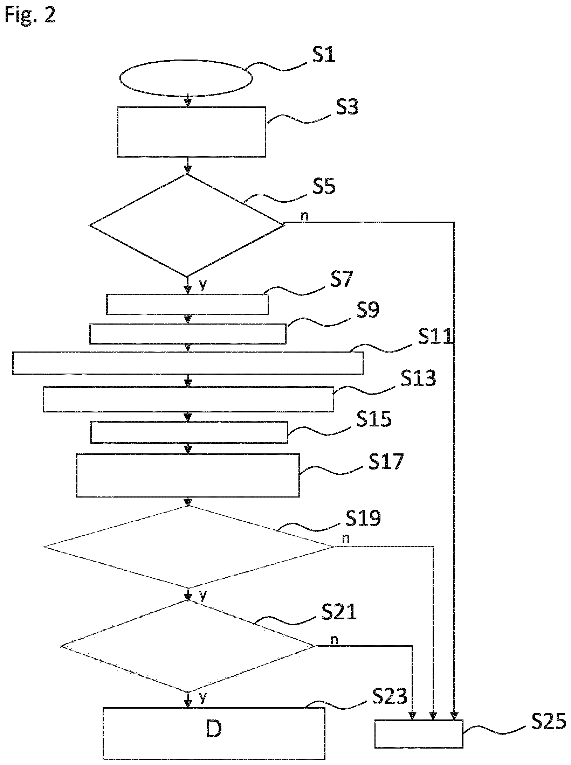

Im Folgenden wird ein Computerprogramm zum Betreiben der Sonde anhand des Ablaufdiagramms der Figur 2 sowie im Zusammenhang mit dem dargestellten Verlauf der Figur 1 näher erläutert, das ausgebildet ist, bei seiner Ausführung auf einer Datenverarbeitungsvorrichtung ein Verfahren gemäß dem allgemeinen Teil der Beschreibung durchzuführen. Insbesondere dient das Programm der Eigendiagnose der Sonde.

Das Programm wird in einem Schritt S1 gestartet, in dem beispielsweise Variablen initialisiert werden. Zu diesem Zeitpunkt kann der Verlauf der Referenzspannung Vref beispielsweise bereits vorliegen. Alternativ kann das Programm beispielsweise in Echtzeit ausgeführt werden.

In einem Schritt S3 wird der erste Zeitpunkt T1 ermittelt, zu dem der an der Referenzzelle anliegende Pumpstrom angeschaltet ist. Der erste Zeitpunkt T1 entspricht insbesondere einem Startzeitpunkt der Eigendiagnose der Sonde. Der erste Zeitpunkt T1 kann beispielsweise abhängig von einem Motor-Betriebszustand gewählt werden. Beispielsweise erfolgt die Eigendiagnose in einem der Motor-Betriebszustände Teillast, Schub, Nachlauf oder λ=1 Betriebspunkt. Diese Betriebszustände sind besonders geeignet zur Eigendiagnose der Sonde, da Schwankungen der Referenzspannung Vref im Wesentlichen lediglich von dem Pumpstrom abhängen.

Ferner wird der zweite Zeitpunkt T2 ermittelt, welcher zeitlich beabstandet ist um eine Zeitspanne korrespondierend zu einem vorgegebenen ersten Zeitfenster F1 (vergleiche Figur 1). Das vorgegebene erste Zeitfenster F1 beträgt beispielsweise zwischen 0,2 s und 2 s. Innerhalb dieses vorgegebenen ersten Zeitfensters F1, also zwischen dem ersten Zeitpunkt T1 und dem zweiten Zeitpunkt T2 wird ein Mittelwert sowie eine Standardabweichung der Referenzspannung Vref ermittelt. Der hierbei ermittelte Mittelwert wird einem ersten Spannungskennwert Vref1 (vergleiche Figur 1) zugewiesen. Das Programm wird anschließend in einem Schritt S5 fortgesetzt.

In dem Schritt S5 wird überprüft, ob die in dem Schritt S3 ermittelte Standardabweichung innerhalb des vorgegebenen ersten Zeitfensters F1 innerhalb einer vorgegebenen Grenze liegt, einen vorgegebenen ersten Schwellwert also nicht überschreitet. Dies dient insbesondere einer Überprüfung, ob die gemessene Referenzspannung innerhalb des vorgegebenen ersten Zeitfensters F1 stabil ist, bevor der Pumpstrom anschließend abgeschaltet wird. Im Falle, dass der vorgegebene erste Schwellwert nicht überschritten wird, wird das Programm in einem Schritt S7 fortgesetzt. Anderenfalls wird das Programm in einem Schritt S25 fortgesetzt.

In dem Schritt S7 wird der Pumpstrom korrespondierend zu dem zweiten Zeitpunkt T2 abgeschaltet. Das Programm wird anschließend in einem Schritt S9 fortgesetzt.

In dem Schritt S9 wird eine Wartephase für eine vorgegebene erste Zeitspanne W1 (vergleiche Figur 1) durchgeführt. Die vorgegebene erste Zeitspanne W1 erstreckt sich dabei von dem zweiten Zeitpunkt T2 hin zu dem dritten Zeitpunkt T3. Dies ermöglicht ein Ausschwingen der Referenzspannung Vref. Ab dem dritten Zeitpunkt T3 ist mit einer stabilen Referenzspannung Vref zu rechnen. Die vorgegebene erste Zeitspanne W1 beträgt hierzu beispielsweise zwischen 0,5 s und 1 s. Das Programm wird anschließend in einem Schritt S11 fortgesetzt.

In dem Schritt S11 wird der dritte Zeitpunkt T3 ermittelt, zu dem der an der Referenzzelle anliegende Pumpstrom abgeschaltet ist. Ferner wird der vierte Zeitpunkt T4 ermittelt, welcher zeitlich auf den dritten Zeitpunkt T3 folgt und zeitlich beab-standet ist um eine Zeitspanne korrespondierend zu einem vorgegebenen zweiten Zeitfenster F2 (vergleiche Figur 1). Das vorgegebene zweite Zeitfenster F2 beträgt beispielsweise zwischen 0,2 s und 2 s. Innerhalb dieses vorgegebenen zweiten Zeitfensters F2, also zwischen dem dritten Zeitpunkt T3 und dem vierten Zeitpunkt T4 wird wiederum ein Mittelwert sowie eine Standardabweichung der Referenzspannung Vref ermittelt. Der hierbei ermittelte Mittelwert wird einem zweiten Spannungskennwert Vref2 (vergleiche Figur 1) zugewiesen. Das Programm wird anschließend in einem Schritt S13 fortgesetzt.

In dem Schritt S13 wird der Pumpstrom korrespondierend zu dem vierten Zeitpunkt T4 angeschaltet. Das Programm wird anschließend in einem Schritt S15 fortgesetzt.

In dem Schritt S15 wird eine Wartephase für eine vorgegebene zweite Zeitspanne W2 (vergleiche Figur 1) durchgeführt. Die vorgegebene zweite Zeitspanne W2 erstreckt sich dabei von dem vierten Zeitpunkt T4 hin zu dem fünften Zeitpunkt T5. Dies ermöglicht ein Ausschwingen der Referenzspannung Vref. Ab dem fünften Zeitpunkt T5 ist mit einer stabilen Referenzspannung Vref zu rechnen. Die vorgegebene zweite Zeitspanne W2 beträgt hierzu beispielsweise zwischen 0,5 s und 1 s. Die vorgegebene zweite Zeitspanne W2 ist dabei insbesondere größer als die erste Zeitspanne W1. Das Programm wird anschließend in einem Schritt S17 fortgesetzt.

In dem Schritt S17 wird der sechste Zeitpunkt T6 ermittelt, zu dem der an der Referenzzelle anliegende Pumpstrom angeschaltet ist. Der sechste Zeitpunkt T6 folgt dabei zeitlich auf den fünften Zeitpunkt T5. Der sechste Zeitpunkt T6 ist dabei insbesondere um ein vorgegebenes drittes Zeitfenster F3 (vergleiche Figur 1) zeitlich beabstandet von dem fünften Zeitpunkt T5. Das vorgegebene dritte Zeitfenster F3 beträgt beispielsweise zwischen 0,2 s und 2 s. Innerhalb dieses vorgegebenen dritten Zeitfensters F3, also zwischen dem fünften Zeitpunkt T5 und dem sechsten Zeitpunkt T6 wird wiederum ein Mittelwert sowie eine Standardabweichung der Referenzspannung Vref ermittelt. Der hierbei ermittelte Mittelwert wird einem dritten Spannungskennwert Vref3 (vergleiche Figur 1) zugewiesen. Das Programm wird anschließend in einem Schritt S19 fortgesetzt.

In dem Schritt S19 überprüft, ob die in dem Schritt S17 ermittelte Standardabweichung innerhalb des vorgegebenen dritten Zeitfensters F3 innerhalb einer vorgegebenen Grenze liegt, einen vorgegebenen zweiten Schwellwert also nicht überschreitet. Dieser vorgegebene zweite Schwellwert kann beispielsweise mit dem vorgegebenen ersten Schwellwert übereinstimmen. Dies dient insbesondere einer Überprüfung, ob die gemessene Referenzspannung innerhalb des vorgegebenen dritten Zeitfensters F3 stabil ist nachdem der Pumpstrom angeschaltet wurde. Im Falle, dass der vorgegebene zweite Schwellwert nicht überschritten wird, wird das Programm in einem Schritt S21 fortgesetzt. Anderenfalls wird das Programm in dem Schritt S25 fortgesetzt. In dem Schritt S21 wird überprüft, ob eine Differenz zwischen dem ersten Spannungskennwert Vref1 und dem dritten Spannungskennwert Vref3 innerhalb einer vorgegebenen Grenze liegt, einen vorgegebenen dritten Schwellwert also nicht überschreitet. Dies dient insbesondere einer Verifizierung, ob die gemessene Referenzspannung Vref zwischen dem ersten Zeitpunkt T1 und dem sechsten Zeitpunkt T6 repräsentativ sind für eine gültige Diagnose. Im Falle, dass der vorgegebene dritte Schwellwert nicht überschritten wird, wird das Programm in einem Schritt S23 fortgesetzt. Anderenfalls wird das Programm in dem Schritt S25 fortgesetzt.

In dem Schritt S23 wird eine Differenz ΔVref zwischen dem ersten Spannungskennwert Vref1 und dem zweiten Spannungskennwert Vref2 ermittelt. Abhängig von dieser Differenz ΔVref wird ein Diagnosekennwert D ermittelt. Der Diagnosekennwert D ist repräsentativ für die Impedanz der Referenzzelle. Diese kann eine Abweichung der durch die Sonde erfassten Stickoxidkonzentration im Verbrennungsabgas bedingen. Der Diagnosekennwert D ist folglich repräsentativ für einen Fehler der Ermittlung der Stickoxidkonzentration. Näherungsweise besteht hier ein linearer Zusammenhang. Beispielsweise erfolgt in diesem Zusammenhang ein Ermitteln dieses Fehlers als Funktion der Differenz ΔVref zwischen dem ersten Spannungskennwert Vref1 und dem zweiten Spannungskennwert Vref2. Ein aufgrund einer zu großen Impedanz der Referenzzelle bedingter Spannungsversatz erfährt dabei beispielsweise eine Bewertung, die als Diagnoseergebnis in den Diagnosekennwert D einfließt. Der Diagnosekennwert D kann insbesondere ausgegeben werden, beispielsweise auf einem Bussystem des Fahrzeugs wie dem CAN-Bus. Das Diagnoseergebnis kann entweder einen binären Wert annehmen wie "gut" oder "schlecht", oder aber einen kontinuierlichen Prozentwert, welcher eine erwartete Abweichung von einem kalibrierten Zustand der Sonde angibt. Dies ermöglicht in vorteilhafter Weise, dass ein Motorsteuergerät den Zustand der Sonde im Feld detektieren kann, also im Betrieb des Fahrzeugs und ohne dass hierfür eine Werkstatt aufgesucht werden muss. Hierbei kann insbesondere festgestellt werden, ob eine Messgenauigkeit der Sonde noch ausreicht, um eine Abgasanlage des Fahrzeugs zu steuern.

Optional wird ferner eine Kompensation der Sollwerte der Nernstspannungen vorgenommen, so dass die Stickoxidkonzentration auch bei Alterung der Sonde weiterhin präzise und zuverlässig ermittelt werden kann. Dabei wird aus dem Diagnoseergebnis ein Korrektur-Parameter ermittelt, der es erlaubt, korrekte Betriebspunkte der Sonde einzustellen und somit die Impedanzveränderung über Alterung oder Veränderung zu kompensieren. Das Programm wird anschließend beendet.

In dem Schritt S25 wird die Eigendiagnose abgebrochen. Das Programm wird anschließend beendet.

Alternativ zu dem in Figur 2 dargestellten Ablaufdiagramm kann beispielsweise auf die Schritte S13 bis S21 verzichtet werden, wenn anderweitig sichergestellt werden kann, dass eine gültige Eigendiagnose vorliegt. Dies ist beispielsweise der Fall, wenn die Eigendiagnose durch ein Steuergerät initiiert wird, welchem zusätzlich Informationen über den Motor-Betriebszustand bereitgestellt werden. In diesem Fall kann sichergestellt werden, dass sich der Motor-Betriebszustand zwischen dem ersten Zeitpunkt T1 und dem vierten Zeitpunkt T4 nicht ändert, bzw. die Eigendiagnose anderenfalls abgebrochen wird. Analog hierzu könnte alternativ auf die Schritte S3 bis S7 verzichtet werden, wenn durch das Steuergerät sichergestellt ist, dass der Motor-Betriebszustand zwischen dem dritten Zeitpunkt T3 und dem sechsten Zeitpunkt T6 unverändert ist, bzw. die Eigendiagnose anderenfalls abgebrochen wird.

Die in dem Ausführungsbeispiel genannte Sonde ist insbesondere eine Sickoxidsonde. In anderen Ausführungsbeispielen kann es sich ebenso um eine Lambdasonde zur Erfassung einer Sauerstoffkonzentration handeln.

## Patentansprüche

1. Verfahren zum Durchführen einer Eigendiagnose einer Stickoxidsonde oder Lambdasonde mit einer gepumpten Referenzzelle, bei dem
- ein erster Zeitpunkt (T1) ermittelt wird, an dem ein an der Referenzzelle anliegender pulsweiten-modulierter Pumpstrom angeschaltet ist und sich die Referenzzelle in einem gepumpten Betriebszustand befindet, wobei
- abhängig von dem ersten Zeitpunkt (T1) zumindest ein erster Spannungskennwert (Vref1) innerhalb eines vorgegebenen ersten Zeitfensters (F1) ermittelt wird, der repräsentativ ist für eine an der Referenzzelle anliegende Spannung, wobei der erste Spannungskennwert (Vref1) während einer Ausschaltzeit des pulsweiten-modulierten Stroms ermittelt wird,
- der an der Referenzzelle anliegende pulsweiten-modulierte Pumpstrom an einem zweiten Zeitpunkt (T2) abgeschaltet wird,
- ein dritter Zeitpunkt (T3) ermittelt wird, an dem ein an der Referenzzelle anliegender pulsweiten-modulierter Pumpstrom abgeschaltet ist und sich die Referenzzelle in einem ungepumpten Betriebszustand befindet, wobei
- der dritte Zeitpunkt (T3) zeitlich auf den zweiten Zeitpunkt (T2) folgt,
- abhängig von dem dritten Zeitpunkt (T3) zumindest ein zweiter Spannungskennwert (Vref2) innerhalb eines vorgegebenen zweiten Zeitfensters (F2) ermittelt wird, der repräsentativ ist für die an der Referenzzelle anliegende Spannung, und
- abhängig von einer Differenz (ΔVref) zwischen dem zumindest einen ersten Spannungskennwert (Vref1) und dem zumindest einen zweiten Spannungskennwert (Vref2) ein Diagnosekennwert (D) ermittelt wird, der repräsentativ ist für einen Fehler der Ermittlung der Stickoxidkonzentration.

2. Verfahren nach Anspruch 1, bei dem zwischen dem zweiten Zeitpunkt (T2) und dem dritten Zeitpunkt (T3) eine Wartephase für eine vorgegebene erste Zeitspanne (W1) durchgeführt wird.

3. Verfahren nach Anspruch 1 oder 2, bei dem der an der Referenzzelle anliegende pulsweiten-modulierte Pumpstrom an einem vierten Zeitpunkt (T4) angeschaltet wird, wobei der vierte Zeitpunkt (T4) zeitlich auf den dritten Zeitpunkt (T3) folgt.

4. Verfahren nach einem der vorstehenden Ansprüche, bei dem
- ein fünfter Zeitpunkt (T5) ermittelt wird, an dem ein ander Referenzzelle anliegender pulsweiten-modulierter Pumpstrom angeschaltet ist und sich die Referenzzelle in einem gepumpten Betriebszustand befindet, wobei der fünfte Zeitpunkt (T5) zeitlich auf den vierten Zeitpunkt (T4) folgt, und
- abhängig von dem fünften Zeitpunkt (T5) zumindest ein dritter Spannungskennwert (Vref3) innerhalb eines vorgegebenen dritten Zeitfensters (F3) ermittelt wird, der repräsentativ ist für die an der Referenzzelle anliegende Spannung.

5. Verfahren nach Anspruch 4, bei dem zwischen dem vierten Zeitpunkt (T4) und dem fünften Zeitpunkt (T5) eine Wartephase für eine vorgegebene zweite Zeitspanne (W2) durchgeführt wird.

6. Verfahren nach einem der vorstehenden Ansprüche 4 oder 5, bei dem der Diagnosekennwert (D) abhängig von dem zumindest einen dritten Spannungskennwert (Vref3) ermittelt wird.

7. Verfahren nach einem der vorstehenden Ansprüche, bei dem ein Ermitteln des Diagnosekennwerts (D) eine Ermittlung eines Mittelwerts abhängig von den jeweiligen Spannungskennwerten (Vref1, Vref2, Vref3) umfasst.

8. Verfahren nach einem der vorstehenden Ansprüche, bei dem ein Ermitteln des Diagnosekennwerts (D) eine Ermittlung einer Standardabweichung abhängig von den jeweiligen Spannungskennwerten (Vref1, Vref2, Vref3) umfasst.

9. Verfahren nach einem der vorstehenden Ansprüche, wobei
- die Sonde eine Messzelle umfasst,
- abhängig von dem Diagnosekennwert (D) ein Sollspannungskennwert ermittelt wird, der repräsentativ ist für eine an der Messzelle anliegende Spannung zur Messung einer Gaskonzentration eines der Sonde zugeführten Gasgemischs, und
- ein an der Messzelle anliegender Strom derart gesteuert wird, dass sich die an der Messzelle anliegende Spannung auf den Sollspannungskennwert einstellt.

10. Verfahren nach einem der vorstehenden Ansprüche, wobei
- die Sonde als Sauerstoffsensor ausgebildet ist oder einen solchen umfasst, und
- abhängig von dem Diagnosekennwert (D) eine Sauerstoffkonzentration eines dem Sauerstoffsensor zugeführten Gasgemischs ermittelt wird.

11. Verfahren nach einem der vorstehenden Ansprüche, wobei
- die Sonde als Stickoxidsensor in einem Abgastrakt einer Verbrennungsmaschine angeordnet ist,
- abhängig von dem Diagnosekennwert (D) eine Stickoxidkonzentration eines dem Stickoxidsensor zugeführten Gasgemischs ermittelt wird, und
- ein Ermitteln des ersten Zeitpunkts (T1) abhängig von einem Betriebszustand der Verbrennungsmaschine durchgeführt wird.

## Claims

1. Method for performing a self-diagnosis of a nitrogen oxide probe or a lambda probe with a pumped reference cell, wherein
- a first point in time (T1) is determined at which a pulse width modulated pump current applied to the reference cell is turned on and the reference cell is in a pumped operating state, wherein
- depending on the first point in time (T1) at least one first voltage characteristic value (Vref1) that is representative of a voltage applied to the reference cell is determined within a predetermined first time window (F1), wherein the first voltage characteristic value (Vref1) is determined during a switched-off time of the pulse width modulated current,
- the pulse width modulated pump current applied to the reference cell is turned off at a second point in time (T2),
- a third point in time (T3) is determined at which a pulse width modulated pump current applied to the reference cell is turned off and the reference cell is in an unpumped operating state, wherein
- the third point in time (T3) is after the second point in time (T2),
- depending on the third point in time (T3) at least one second voltage characteristic value (Vref2) that is representative of the voltage applied to the reference cell is determined within a predetermined second time window (F2), and
- depending on a difference (ΔVref) between the at least one first voltage characteristic value (Vref1) and the at least one second voltage characteristic value (Vref2), a diagnostic characteristic value (D) is determined that is representative of an error in the determination of the oxides of nitrogen concentration.

2. Method according to claim 1, wherein a wait phase for a predetermined first period of time (W1) is carried out between the second point in time (T2) and the third point in time (T3).

3. Method according to Claim 1 or 2, wherein the pulse width modulated pump current applied to the reference cell is turned on at a fourth point in time (T4), wherein the fourth point in time (T4) is after the third point in time (T3).

4. Method according to any one of the preceding claims, wherein
- a fifth point in time (T5) is determined at which a pulse width modulated pump current applied to the reference cell is turned on and the reference cell is in a pumped operating state, wherein the fifth point in time (T5) is after the fourth point in time (T4), and
- depending on the fifth point in time (T5), at least one third voltage characteristic value (Vref3) that is representative of the voltage applied to the reference cell is determined within a predetermined third time window (F3).

5. Method according to Claim 4, wherein between the fourth point in time (T4) and the fifth point in time (T5) a wait phase is carried out for a predetermined second period of time (W2).

6. Method according to any one of the preceding Claims 4 or 5, wherein the diagnostic characteristic value (D) is determined as a function of the at least one third voltage characteristic value (Vref3).

7. Method according to any one of the preceding claims, wherein a determination of the diagnostic characteristic value (D) comprises determining an average value depending on the respective voltage characteristic values (Vref1, Vref2, Vref3).

8. Method according to any one of the preceding claims, wherein a determination of the diagnostic characteristic value (D) comprises determining a standard deviation depending on the respective voltage characteristic values (Vref1, Vref2, Vref3).

9. Method according to any one of the preceding claims, wherein
- the probe comprises a measurement cell,
- a target voltage characteristic value that is representative of a voltage applied to the measurement cell for measuring a gas concentration of a gas mixture delivered to the probe is determined as a function of the diagnostic characteristic value (D), and
- a current applied to the measurement cell is controlled such that the voltage applied to the measurement cell is set to the target voltage characteristic value.

10. Method according to any one of the preceding claims, wherein
- the probe is implemented as or comprises an oxygen sensor, and
- an oxygen concentration of a gas mixture delivered to the oxygen sensor is determined as a function of the diagnostic characteristic value (D).

11. Method according to any one of the preceding claims, wherein
- the probe is disposed as a nitrogen oxide sensor in an exhaust duct of an internal combustion engine,
- a nitrogen oxide concentration of a gas mixture delivered to the nitrogen oxide sensor is determined as a function of the diagnostic characteristic value (D), and
- a determination of the first point in time (T1) is carried out depending on an operating state of the internal combustion engine.

## Revendications

1. Procédé de réalisation d'un autodiagnostic d'une sonde d'oxyde d'azote ou d'une sonde lambda comprenant une cellule de référence pompée, selon lequel
- un premier instant (T1) est déterminé, auquel un courant de pompage modulé en largeur d'impulsions appliqué à la cellule de référence est mis en circuit et la cellule de référence se trouve dans un état opérationnel pompé,
- au moins une première valeur caractéristique de tension (Vref1) étant déterminée en fonction du premier instant (T1) à l'intérieur d'une première fenêtre temporelle (F1) prédéfinie, laquelle est représentative d'une tension présente au niveau de la cellule de référence, la première valeur caractéristique de tension (Vref1) étant déterminée pendant un temps d'arrêt du courant modulé en largeur d'impulsions,
- le courant de pompage modulé en largeur d'impulsions appliqué à la cellule de référence est mis hors circuit à un deuxième instant (T2),
- un troisième instant (T3) est déterminé, auquel un courant de pompage modulé en largeur d'impulsions appliqué à la cellule de référence est mis hors circuit et la cellule de référence se trouve dans un état opérationnel non pompé,
- le troisième instant (T3) suivant chronologiquement le deuxième instant (T2),
- en fonction du troisième instant (T3), au moins une deuxième valeur caractéristique de tension (Vref2) est déterminée à l'intérieur d'une deuxième fenêtre temporelle (F2) prédéfinie, laquelle est représentative de la tension présente au niveau de la cellule de référence, et
- une valeur caractéristique de diagnostic (D) est déterminée en fonction d'une différence (ΔVref) entre l'au moins une première valeur caractéristique de tension (Vref1) et l'au moins une deuxième valeur caractéristique de tension (Vref2), laquelle est représentative d'une erreur de détermination de la concentration d'oxyde d'azote.

2. Procédé selon la revendication 1, selon lequel une phase d'attente est effectuée entre le deuxième instant (T2) et le troisième instant (T3) pendant un premier intervalle de temps (W1) prédéfini.

3. Procédé selon la revendication 1 ou 2, selon lequel le courant de pompage modulé en largeur d'impulsions appliqué à la cellule de référence est mis en circuit à un quatrième instant (T4), le quatrième instant (T4) suivant chronologiquement le troisième instant (T3).

4. Procédé selon l'une des revendications précédentes, selon lequel
- un cinquième instant (T5) est déterminé, auquel un courant de pompage modulé en largeur d'impulsions appliqué à la cellule de référence est mis en circuit et la cellule de référence se trouve dans un état opérationnel pompé, le cinquième instant (T5) suivant chronologiquement le quatrième instant (T4),
- en fonction du cinquième instant (T5), au moins une troisième valeur caractéristique de tension (Vref3) est déterminée à l'intérieur d'une troisième fenêtre temporelle (F3) prédéfinie, laquelle est représentative de la tension présente au niveau de la cellule de référence.

5. Procédé selon la revendication 4, selon lequel une phase d'attente est effectuée entre le quatrième instant (T4) et le cinquième instant (T5) pendant un deuxième intervalle de temps (W2) prédéfini.

6. Procédé selon l'une des revendications précédentes 4 ou 5, selon lequel la valeur caractéristique de diagnostic (D) est déterminée en fonction de l'au moins une troisième valeur caractéristique de tension (Vref3).

7. Procédé selon l'une des revendications précédentes, selon lequel une détermination de la valeur caractéristique de diagnostic (D) comprend une détermination d'une valeur moyenne dépendante des valeurs caractéristiques de tension (Vref1, Vref2, Vref3) respectives.

8. Procédé selon l'une des revendications précédentes, selon lequel une détermination de la valeur caractéristique de diagnostic (D) comprend une détermination d'un écart type dépendant des valeurs caractéristiques de tension (Vref1, Vref2, Vref3) respectives.

9. Procédé selon l'une des revendications précédentes,
- la sonde comprenant une cellule de mesure,
- une valeur caractéristique de tension de consigne étant déterminée en fonction de la valeur caractéristique de diagnostic (D), laquelle est représentative d'une tension présente au niveau de la cellule de mesure en vue de mesurer une concentration de gaz d'un mélange gazeux acheminé à la sonde, et
- un courant appliqué à la cellule de mesure étant commandé de telle sorte que la tension présente au niveau de la cellule de mesure se règle à la valeur caractéristique de tension de consigne.

10. Procédé selon l'une des revendications précédentes,
- la sonde étant réalisée sous la forme d'un capteur d'oxygène ou en comprenant un, et
- une concentration d'oxygène d'un mélange gazeux acheminé au capteur d'oxygène étant déterminée en fonction de la valeur caractéristique de diagnostic (D).

11. Procédé selon l'une des revendications précédentes,
- la sonde, en tant que capteur d'oxyde d'azote, étant disposée dans un trajet de gaz d'échappement d'un moteur à combustion interne,
- une concentration d'oxyde d'azote d'un mélange gazeux acheminé au capteur d'oxyde d'azote étant déterminée en fonction de la valeur caractéristique de diagnostic (D), et
- une détermination du premier instant (T1) étant effectuée en fonction d'un état opérationnel du moteur à combustion interne.
